# EUROPEAN PATENT SPECIFICATION

(11) **EP 4 536 455 B1**
(45) Date of publication and mention of the grant of the patent: **25.02.2026**
(21) Application number: 23731344.0
(22) Date of filing: 05.06.2023
(51) Int. Cl.: B28C 7/02, B28C 5/42, G01N 33/38, G06N 3/08

(54) **SLUMP ESTIMATION FOR CONCRETE MIXERS**
SETZMASSSCHÄTZUNG FÜR BETONMISCHER
ESTIMATION D'AFFAISSEMENT POUR MÉLANGEURS DE BÉTON

(30) Priority: 06.06.2022 GB 202208264
(43) Date of publication of application: 16.04.2025
(73) Proprietor: Total Vehicle Solutions Group Limited, Wisbech Cambridgeshire PE14 0SB (GB)
(72) Inventor: WALKER, Kevin, Wisbech Cambridgeshire PE14 0SB (GB); DAVIES, John Darren, Buckingham Buckinghamshire MK18 1UL (GB); HICKS, Matthew, Buckingham Buckinghamshire MK18 1UL (GB)
(74) Representative: Varley, James Richard
(86) International application number: PCT/GB2023/051466
(87) International publication number: WO 2023/237862

(56) References cited:
- CA-A1- 3 068 681
- US-B1- 11 312 039

## Description

### Field

This specification describes systems, apparatus and methods for measuring the slump of a concrete mix using one or more machine-learning models. This specification further describes systems, apparatus and methods for monitoring concrete build-up in a mixing drum.

### Background

Slump refers to the viscosity of the concrete mix - higher slump is lower viscosity (and vice versa). For some construction jobs, slump is a crucial factor (e.g. kerb extrusions) and a sample of the concrete is tested before it is fully discharged from the drum. Slump testing can cause higher rejection rates with up to 70% of deliveries rejected (returned to depot) when slump testing is employed. It is therefore beneficial to ensure that slump meets requirements prior to delivery.

There are some slump measurement systems that use sensors inside the drum to measure batch quality. However, these sensors are an indirect measurement, relatively expensive and requiring regular maintenance as they are inside the drum.

Furthermore, concrete mixing drums also suffer from build-up of dried concrete on the inside wall. Even a 1mm deposit over the entire wall may add approximately 100 kg of weight to the drum, which can negatively affect its performance and increase the energy consumption of the vehicle carrying the drum. This is an especially important consideration in electrically powered vehicles. Furthermore, in weight-limited situations, the build-up reduces the payload of concrete that can be delivered.

Typically, operators carry out drum build up removal periodically (typically 6 months). This requires the concrete mixer to be taken off the road for a lengthy period of time. Consequently, a better way of assessing and quantifying build up and rotational drag is required so that operators can assess the optimum time for taking the vehicle off the road.

CA 3068681 A1 discloses a method for predicting a slump value of a concrete in a mixing drum of a concrete mixer in accordance with the preamble of claim 1 and a method for monitoring concrete build-up in a mixing drum in accordance with the preamble of claim 7, more particularly it discloses a mixer vehicle that includes a mixer drum, a first acceleration sensor, a second acceleration sensor, and a controller. The first acceleration sensor is configured to produce first acceleration signals and the second acceleration sensor is configured to measure accelerations within the mixer drum to produce second acceleration signals. The controller is configured to receive the first acceleration signals from the first acceleration sensor and second acceleration signals from the second acceleration sensor. The controller is further configured to determine a presence of material within the mixer drum based on the first acceleration signals and the second acceleration signals. The controller is further configured to determine one or more properties of the material within the mixer drum based on the first acceleration signals and the second acceleration signals.

US 11312039 B1 discloses a system for a concrete mixer having a drum receiving fresh concrete therein. The system generally has: a sensor measuring a set of measurand values indicative of a measurand associated with at least one of the fresh concrete, the drum and components of the concrete mixer; and a controller communicatively coupled to the sensor, the controller performing the steps of: accessing the set of measurand values generated by the sensor; using a trained data processing engine stored on the non-transitory memory, at least one of determining a property value indicative of a property of the fresh concrete, determining a parameter value indicative of a parameter of the drum, and determining that the set of measurand values are indicative of some operating conditions of the concrete mixer; and outputting a signal based on said determining.

### Summary

The invention is defined in the appended claims.

According to a first aspect of this specification, there is described a method for predicting a slump value of a concrete mix in a mixing drum of a concrete mixer. The method comprises: receiving, from one or more sensors, motor data comprising a state of a motor driving the mixing drum, gearbox data comprising a state of a gearbox arranged between the motor and the mixing drum, and load data relating to a mass of the concrete mix in the mixing drum; determining, by a machine-learned rotation power model, an initial rotation power of the motor from the motor data; determining, by a gearbox efficiency model, a gearbox efficiency from the gearbox data; determining an adjusted rotation power by adjusting the initial rotation power of the motor of the motor based on the gearbox efficiency; and determining, by a slump prediction model, an estimated slump value of the concrete mix in the mixing drum from the load data and the adjusted rotation power.

The method may further comprise performing a zeroing procedure, the zeroing procedure comprising: for each of a plurality of rotation speeds: rotating the mixing drum without the concrete mix present; and determining, using the machine-learned rotation power model, a rotation power for the mixing drum at the rotation speed; and determining an empty drum rotation profile based on the respective determined rotation powers for each rotation speed.

The method may further comprise adjusting the initial rotation power of the motor based on the empty drum rotation profile prior to input into the slump prediction model and/or comparing the empty drum rotation profile to a baseline rotation profile to assess drum build up.

The gearbox efficiency model and/or slump prediction model may comprise one or more of: a machine-learned model; a neural network; a lookup table; or a control surface.

The method may further comprise: receiving a target slump value for the concrete mix; and determining, by a hydration model, an amount of water and/or plasticizer to add to the concrete mix to achieve the target slump based at least in part on the target slump value, the estimated slump value and the load data. The method may further comprise causing the concrete mixer to add the determined amount of water and/or plasticizer to the concrete mix.

According to a further aspect of this specification, there is described a method for monitoring concrete build up in a mixing drum, the method comprising: receiving, from one or more sensors, drum mass data comprising a current mass of the mixing drum when empty; for each of a plurality of drum rotation speeds: receiving, from one or more sensors, motor data comprising a state a motor driving the mixing drum and gearbox data comprising a state of a gearbox arranged between the motor and the mixing drum; determining, by a machine-learned rotation power model, an initial rotation power of the motor from the motor data; determining, by a gearbox efficiency model, a gearbox efficiency from the gearbox data; and determining an adjusted rotation power for the drum rotation speed by adjusting the initial rotation power of the motor of the motor based on the gearbox efficiency; determining, using a build-up model, a drum build-up state of the concrete mixer from the adjusted rotation powers for the plurality of drum rotation speeds.

The drum build-up state may comprises: a rotational drag of the mixing drum; a mass of concrete build-up in the mixing drum; an energy consumption increase associated with concrete build-up in the mixing drum; and/or payload reduction caused by concrete build-up in the mixing drum.

Each aspect may include one or more of the following features, either alone or in combination.

The motor may be an electric motor/ The motor data may comprise: a motor speed; an electrical current and/or electrical voltage supplied to the electric motor; and a temperature of the electric motor.

The motor may be a hydraulic motor. The motor data may comprise: a motor speed; an input fluid pressure; an output fluid pressure; and a fluid temperature.

The gearbox data may comprise: an input torque; a motor or drum speed; a gearbox temperature; and a gearbox fluid level.

The machine-learned rotation power model may comprise a neural network. The neural network may be a fully connected neural network.

According to a further aspect of this specification, there is described a system in accordance with claim 13.

The system may form a part of a concrete mixer truck.

According to a further aspect of this specification, there is described a computer program product comprising computer readable instructions that, when executed by the system a system comprising: a mixing drum coupled to a motor via a gearbox; a plurality of sensors; one or more processors; and a memory, cause the system to perform any of the methods disclosed herein.

The examples described herein may be implemented to realise one or more of the following advantages.

Drum rotation power is a complex calculation relying on more than just hydraulic pressure and drum speed. Changes in hydraulic fluid or e-motor winding temperature can lead to significant differences in power delivery. The use of a machine-learned model rather than a complex physical model can increase the accuracy and speed of drum rotation power calculations for concrete mixers. Furthermore, re-calibrating slump calculations to current conditions is generally time consuming and so an irregular occurrence; it also does not account for the change in performance that may occur after calibration, especially as temperatures change. The use machine learning models trained on a range of parameters including temperature values can alleviate this issue.

### Brief Description of the Drawings

Example implementations will be described by way of reference to the accompanying drawings, in which:
FIG. 1 shows a schematic diagram of a concrete mixing system;
FIG. 2 shows a schematic overview of an example method of measuring the slump of a concrete mix;
FIG. 3 shows a schematic overview of a method of training a machine-learned model;
FIG. 4 shows a flow diagram of an example method of measuring the slump of a concrete mix;
FIG. 5 shows a schematic overview of a method for monitoring a drum of a concrete mixer; and
FIG. 6shows a schematic overview of a computer system.

### Detailed Description

A system comprising an artificial intelligence (AI) model (using machine learning, or deep learning) is used to assess the slump of a drum load in a concrete mixer. The system may optionally have the ability to add water or plasticizer to the concrete mix to adjust the slump, if required. The measurement process is a non-invasive one, which can be carried out on a static vehicle or moving vehicle.

The method is based on the use of a machine-learned model to estimate the rotation power of a motor rotating the drum of a concrete mixer. The rotation power determined by the machine-learned model is then used to assess the slump of the concrete mix in the mixer using a slump-power model also referred to herein as a "slump prediction model"). Since the rotation power of a motor is a complex function of the power supplied to the motor, the motor temperature and the rotation speed, the use of machine-learned model can simplify the determination of the rotation power, while also increasing the accuracy of the determined rotation power, and consequently the slump measurement.

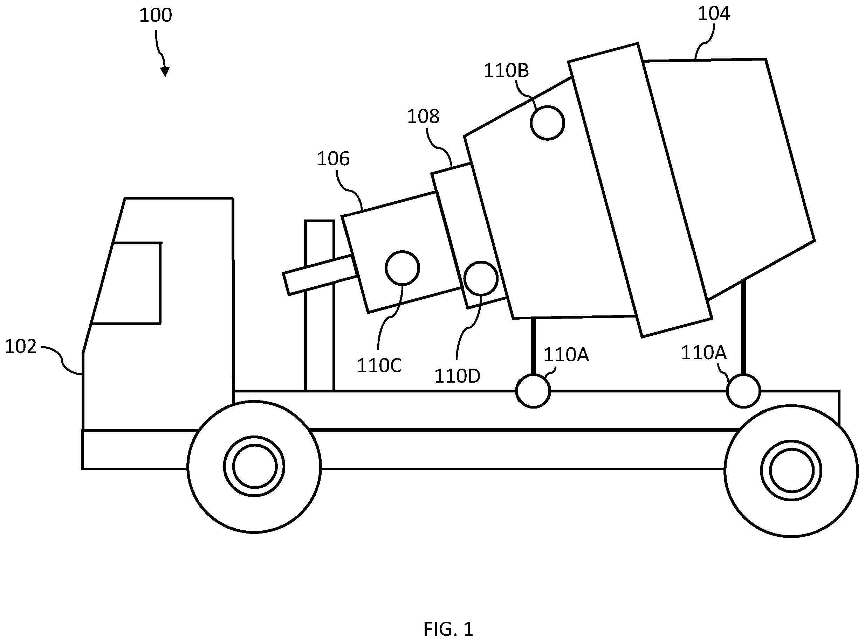

FIG. 1 shows a schematic diagram of an example concrete mixing system 100. In the embodiments shown, the system 100 is part of a concrete mixer truck 102, though it will be appreciated that the system 100 may be applied to other types of concrete mixer.

The system 100 comprises a drum 104 for mixing concrete. The drum 104 is rotatable about a central axis to mix the concrete. The drum 104 may contain internal protrusions (not shown) to aid turning of the concrete mix. A drive motor 106 is coupled to drum via a gearbox 108. The drive motor 106 applies torque to the drum 104 via the gearbox 108 in order to rotate the drum 104. The drive motor 106 may be a bidirectional drive motor, i.e. capable of rotating the drum 104 both clockwise and anticlockwise around the central axis. The drive motor 106 may be a hydraulic or electric motor.

The system 100 further comprises a plurality of sensors 110A-D. The sensors 110A-X are configured to measure properties of the system 100 and/or the concrete mix.

The plurality of sensors may comprise one or more load sensors 110A. The load sensors 110A are configured to measure the load of the concrete mixer, e.g. the mass of the drum 104 plus the mass of the concrete mix in the drum 104. In some embodiments, the one or more load sensors comprises at least two load sensors 110A: a front load sensor arranged to measure the load at the front of the drum 104 (i.e. the end closest to the drum 104 mouth); and a rear load sensor arranged to measure the load at the rear of the drum 104 (i.e. the end furthest from the drum 104 mouth). The load sensors 110A may be zeroed prior to the concrete mix being loaded to account for build-up of dried concrete in the drum.

The plurality of sensors may further comprise a drum temperature sensor 110B configured to measure the temperature of the drum or the concrete in the drum directly. The temperature can be a contact temperature (either internal or external to the drum) or a non-contact temperature measurement. The temperature of the concrete mix can be an important factor in determining the slump of the concrete, since the temperature affects the evaporation rate of moisture in the concrete mix.

The plurality of sensors may further comprise one or more (e.g. a plurality) of motor state sensors 110C configured to measure the state of the motor 106, e.g. the current/voltage supplied to the motor for an electric motor, the input and output hydraulic pressures for a hydraulic motor, the motor temperature, the hydraulic fluid temperature and/or the motor rotation speed. The motor state sensors 110C may further comprise a drum speed sensor, such as an optical encoder or magnetic sensor, though this may alternatively be fitted to the drum itself, when present.

The plurality of sensors may further comprise one or more (e.g. a plurality) of gearbox state sensors 110D configured to measure the state of the gearbox 108, e.g. the input torque/rotation speed to the gearbox, the gearbox temperature, the gearbox fluid level, or the like. Each of the gearbox state sensors 110E may be fitted to the gearbox directly, or to some other part of the system, e.g. the motor for an input torque sensor.

It will be appreciated that there are multiple possible sensors or combinations of sensors that can be arranged to measure the variables required for the methods used herein. As an example, the input torque may be determined using a direct torque measurement via torque sensor on the drive system. Alternatively, the torque can be derived indirectly from other measurements of the motor, such as input current, input and output hydraulic pressures or the like. As another example, the drum speed may be determined from the motor speed and the gearbox ratio, or alternatively measured directly.

The sensors may be connected to an electronic control unit (ECU, not shown) of the concrete mixer. The ECU may use the sensor data to determine properties of the system 100, and to control the other elements of the system.

In some embodiments, the concrete mixer further comprises one or more reservoirs containing water and/or one or more reservoirs containing a plasticizer (not shown). The reservoirs are fluidly connected to the drum 104. Each reservoir may contain a pump for pumping the contents of the reservoir into the drum to hydrate/plasticise the concrete mix. The pump may be configured to receive signals from the ECU of the concrete mixer indicating how much (e.g. a volume) of its contents to transfer to the drum, and pump that quantity of fluid from the reservoir into the drum in response to receiving the signals.

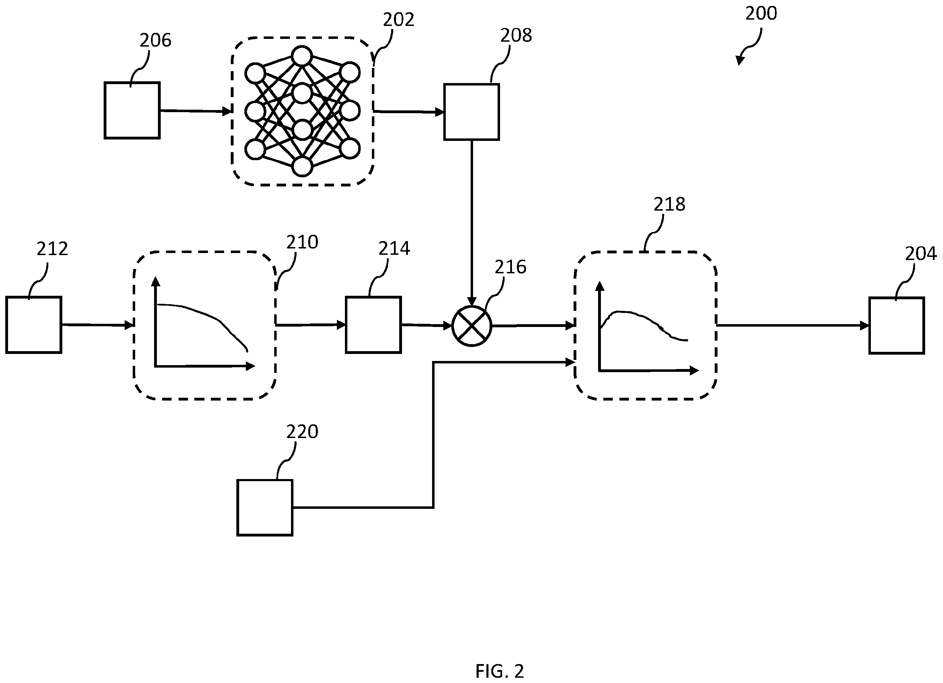

FIG. 2 shows a schematic overview of a method 200 of measuring the slump of a concrete mix. The method may be implemented by one or more computing devices. The computing devices may form part of the system shown in FIG. 1, for example that are part of an electronic control unit (ECU) of the concrete mixer. The method uses a plurality of models 202, 210, 218 to estimate the slump from sensor data obtained from the system.

A machine-learned rotation power model 202 (also referred to herein as a "first machine-learned model") takes as input data 206 relating to a state of a motor driving a drum of a concrete mixer (also referred to herein as "motor data") and processes it to determine an initial estimate of the rotation power 208 of the motor (also referred to herein as an "initial rotation power"). In general, drum rotation power is a complex calculation relying on more than just hydraulic pressure/power supplied to the motor and drum speed. Changes in hydraulic or e-motor temperature can lead to significant differences in power requirements, which are hard to account for using physical models. The use of a machine learned model allows for fast and accurate determination of the rotation power without recourse to the approximations used in physical models.

The motor data 206 may comprise a motor speed, e.g., the current rotational speed of the motor, and/or a load. Where the motor is an electric motor, the motor data 206A may further comprise a current and/or a voltage supplied to the motor, and a motor temperature. Where the motor is a hydraulic motor, the motor data 206A may further comprise an input hydraulic pressure and/or an output hydraulic pressure supplied to the motor, and a hydraulic fluid temperature.

The machine-learned rotation power model 202 may comprise a neural network model. A neural network comprises a plurality of neural network layers, each comprising a plurality of nodes. Each node takes one or more inputs, and determines a node activation value based on a weighted sum of its inputs, where the weights of the sum are learned parameters of the neural network. A non-linear activation function, such as a sigmoid function or ReLu function, is applied to the weighted sum to determine the activation value for the node. Nodes in an input layer of the neural network take as input the input data to the network, with nodes in subsequent layers taking as input one or more outputs from a previous layer. A final layer of the network, known as the output layer provides the neural network output. Layers between the final layer and input layer are referred to as hidden layers.

In some embodiments, the neural network may be a fully connected neural network. A fully connected neural network is a network in which each node in a hidden layer or the final layer takes as input the activation value of every node in the preceding layer. The neural network may, for example, be a relatively shallow neural network, comprising an input layer, and output layer and one or two hidden layers. The neural network may alternatively be a convolutional neural network (CMM) or a recurrent neural network (RNN).

Other types of machine-learned model may alternatively be used. These include, but are not limited to, regression models, support vector machines, genetic models or the like.

A gearbox efficiency model 210 is used to estimate a gearbox efficiency 214 that is used to adjust the initial rotation power to determine an adjusted estimate of the drum rotation power. The gearbox efficiency model 210 takes as input data 212 relating the state of a gearbox coupling the motor to the drum of a concrete mixer (also referred to herein as "gearbox data") and determine an efficiency 214 of the gearbox. The gearbox data 212 may comprise an input toque to the gearbox. The gearbox data 212 may further comprise a gearbox temperature and/or a gearbox fluid level. The gearbox data 212 may comprise a drum speed.

The gearbox efficiency model 210 may be a parametrised curve derived from a physical model or real-world data. Alternatively, the gearbox efficiency model 210 may be a look-up table. Alternatively, the gearbox efficiency model 210 may be a machine-learned model, such as a neural network, as described above in relation to the machine-learned rotation power model 202.

The output of the machine-learned rotation power model 202 (i.e., the rotation power 208 of the motor) and gearbox efficiency model 210 (i.e., the gearbox efficiency 214) may be combined 216 to generate an estimated rotation power value, i.e., the initial rotation power 208 estimated by the machine-learned rotation power model 202 is adjusted based on the gearbox efficiency 214. For example, the initial rotation power 208 may be multiplied by the gearbox efficiency 214 to obtain the rotation power supplied to the drum.

A slump prediction model 218 is used to estimate the slump 204 of the concrete mix from the adjusted rotation power and further input data 220. The further input data may comprise a concrete mix temperature and/or a concrete mix mass (i.e., the load of the mixer). The further input data may also comprise a drum rotation speed. The power required to rotate the drum at a constant speed is a function of slump, volume/mass, and drum geometry, which is captured by the slump prediction model. In some embodiments, the slump prediction model may be drum geometry specific, i.e., each model may have been derived from a mixing drum or simulation of a mixing drum with a particular drum geometry, e.g., length, internal/external diameter, internal protrusions etc.

The slump prediction model 218 may be a parametrised curve derived from a physical model or real-world data. Alternatively, the slump prediction model 218 may be a lookup table. Alternatively, the slump prediction model 218 may be a machine-learned model, such as a neural network, as described above in relation to the machine-learned rotation power model 202.

In some embodiments, the slump prediction 204 is used to determine, by a hydration model (not shown), an amount (e.g., a volume or mass) of water and/or plasticizer that should be added to the concrete mix to achieve a target slump value. The target slump value may be a user input slump value. The hydration model may take as input the slump prediction 204, the target slump and the concrete load (e.g., the mass of the concrete mix), and, in some embodiments, one or more of the sizes of aggregate used in the mix, whether air is entrained or not , and/or other mineral admixtures that are included, and process it to determine the amount of water and/or plasticizer that should be added to the concrete mix.

The some of the variables that may be used in the slump prediction model, such as the size of aggregate used in the mix and/or the mineral admixtures that are included may not be measurable once the mix has been inserted into the mixing drum. These variables may have been decided at the batching plant when first creating the concrete mix. In some embodiments, these variables can be manually input into the control system of the concrete mixer, and stored in a memory therein for use by the hydration model. Alternatively or additionally, these variables may be transmitted from the batching plant to the concrete mixer, for example via a wireless communications network, and stored in the memory of the control system.

Output of the determined amount of water and/or plasticizer may cause the concrete mixer to add the determined amount of water and/or plasticizer to the concrete mix automatically. For example, the output of the determined amount of water and/or plasticizer may cause the ECU of the concrete mixer to transmit instructions to a pump in the concrete mixer to pump the determined amount of water and/or plasticizer from a respective reservoir forming part of the concrete mixer into the drum. Alternatively, the amount of water and/or plasticizer that should be added to the concrete mix may be output via a graphical user interface of the concrete mixer, so that a user can add the water and/or plasticizer manually.

The hydration model may be determined or modified based on experimental data. In general, the model will be a complex function of characteristics across the slump range, e.g. adding 100 gallons/cube (i.e., 456 litres/m³) has different effect on a concrete mix with 20mm slump with coarse aggregate, when compared to a concrete mix with 200mm slump and finer aggregate.

In some implementations, the hydration model may be based on an evaporation model. In general, a concrete mix will start to dry out as soon as it is mixed, with the majority of its drying being down to evaporation. The hydration model may therefore be dependent on the time elapsed since the concrete mix was created. The system may track the time since the concrete mix was formed, and base the determined amount of water/plasticizer required on the duration of time since the concrete mix was created.

For example, in some embodiments the system may maintain a timer that starts counting from the time that the concrete was first mixed or last hydrated. When the hydration model is called, the time from the timer is used as an input to the model. Alternatively, the system may maintain a timestamp for the time that the concrete was mixed/last hydrated. When the hydration model is called, the system may compare a current time to the timestamp associated with the concrete mix to determine the elapsed time to be input into the hydration model.

In some embodiments, a simple hydration model may be used that only uses the elapsed time to determine the amount of water/plasticizer that needs adding. More complex models that account for other variables may alternatively be used. For example, the evaporation rate may also depend on the ambient temperature and/or humidity of the environment of the mix. These variables may be used as additional inputs into the hydration model.

In some embodiments, the system may measure the humidity and/or temperature periodically (e.g., every minute) to determine a record of the conditions the mix has been subject too. This record may be used as input to the hydration model. Use of such a record allows the hydration model to account for variations in the ambient environment, which may result in more accurate predictions about the amount of water/plasticizer that should be added to reach the target slump value.

In some embodiments, a drum zeroing procedure is used to establish the power requirements for rotating an empty drum and to zero the load cells (i.e. the mass sensors) on the drum. This procedure may involve: a) ensuring drum is empty; and then b) rotating drum at a plurality of (constant) speeds and assessing the power required using the machine-learned rotation power model 202. The result is a rotation profile of the empty drum. The zero weight and power data may be stored and used for future calculations. A baseline rotation profile comprising a rotation profile of a drum when new and empty may also be stored as a reference for a new machine.

The first baseline zeroing may be carried out in the factory, and can be stored permanently in the ECU of the concrete mixer. Zeroing may be repeated at intervals during the drum service life (e.g. every 3 months or 6 months) to ensure the system operation is consistent with baseline readings, and to assess drum build up (i.e. concrete deposited to wall of drum).

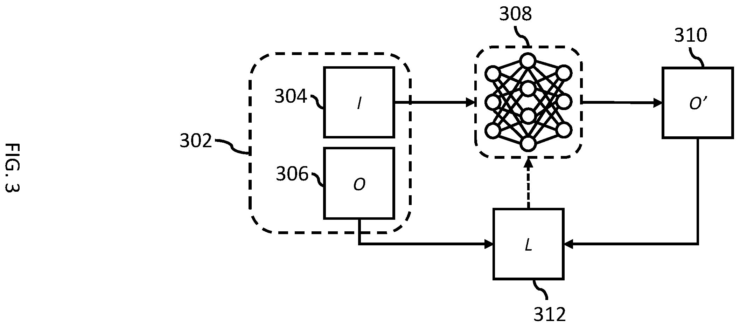

FIG. 3 shows a schematic overview of a method 300 of training a machine-learned model 308. The method may be applied to train the machine-learned rotation power model of FIG.2 and, in embodiments where they are also machine-learned models, the gearbox efficient model and/or the slump prediction model.

Depending on the network being trained, a training sample 302 is obtained from a training dataset. The training sample comprises one or more known inputs 304, I, to the machine-learned model 308 and a corresponding ground truth output 306, O. The training dataset may be generated from real-world examples of the motor, gearbox and/or concrete mixer. Alternatively or additionally, simulated examples from a computer simulation of the motor, gearbox and/or concrete mixer may be used in the training dataset.

For example, for training the rotation power model of FIG. 2, the training data may be generated from a test rig of a motor comprising the motor, one or more high accuracy torque sensors and one or more input sensors (e.g. voltage/current sensors, input/output hydraulic pressure sensors or the like). One or more temperature sensors may also be present to measure the temperature of the motor and/or the temperature of the hydraulic fluid. Operating parameters of the motor (i.e. the parameters to be used as input values to the machine-learned model) are varied, and corresponding output values (i.e. the rotation power) are measured/derived from measurements on the motor to generate the training examples for the training dataset. Each training example comprises measured motor data and a corresponding measured rotation power.

As another example, for training a machine-learned gearbox efficiency model of FIG. 2, each training example may comprise gearbox data (e.g. input torque, speed, temperature, fluid level or the like) and a corresponding gearbox efficiency or output torque. The training data may be determined experimentally using a gearbox tested on a purpose-built rig, using high accuracy torque sensors and varying operation parameters.

As a further example, for training a machine-learned slump prediction model of FIG. 2 the training examples may each comprise known rotation powers, drum temperatures and concrete mix volume/masses, with the ground truth data comprising a corresponding slump of the concrete mix. Training data may be generated experimentally by, for example, placing concrete with a known slump and mass into the concrete mixer and measuring the rotation power required to rotate the drum at a set of drum speeds. The temperatures may also be measured. Alternatively or additionally, training data may be generated from computational fluid dynamics models of the concrete mix in a rotating drum. Note that specific slump prediction models may be trained for each mixing drum geometry, with the model used at inference time depending on the type of mixing drum being used.

It will be appreciated that the training data for the gearbox model and/or slump prediction model may alternatively be used to create respective look up tables and/or control surfaces instead of training a machine-learned model 308.

The known input 304 is input into a machine-learned model 308, which processes the input 304 according to current values of parameters (e.g. weights and biases) of the machine-learned model 308 to generate a candidate output 310, O'. The candidate output 310 is compared to the ground truth output 306, for example using a loss/objective function 312. Based on the comparison, the parameters of the machine-learned model 308 are updated. For example, an optimisation routine, such as stochastic gradient descent, may be applied to the loss/objective function 312 in order to determine the parameter updates. In some implementation, each set of updates may be determined based on a plurality of training examples 302, i.e. a batch of training data.

The loss/objective function 312 may be based on a difference between the candidate output 310 and the ground truth output 306, i.e. O'-O. Examples of such loss functions include, but are not limited to, an L2 or L1 loss.

The training may be iterated until a termination condition is satisfied. The termination condition may be a threshold number of training epochs and/or a threshold performance on a training dataset.

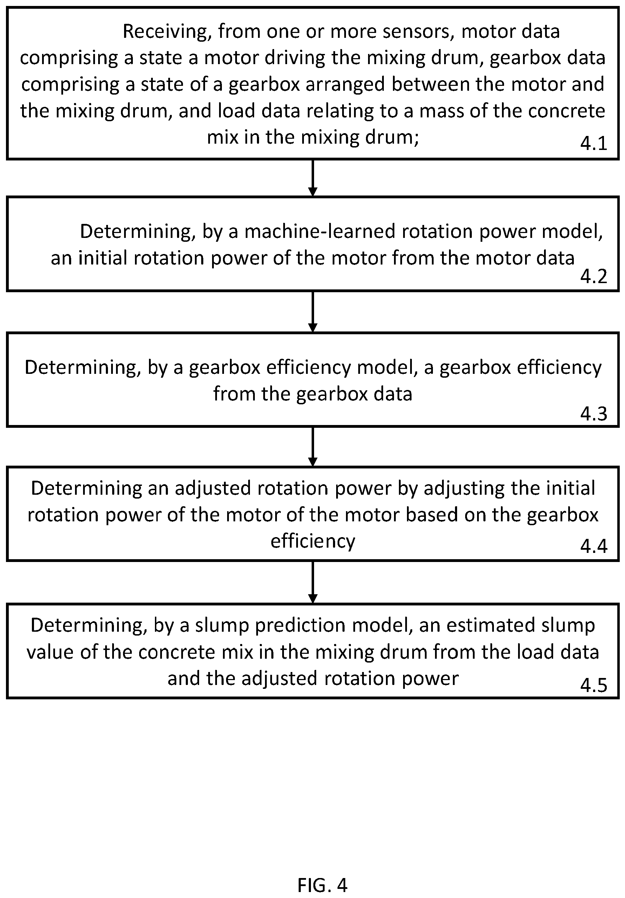

FIG. 4 shows a flow diagram of an example method for estimating the slump of a concrete mix in a concrete mixer. The method may be performed by a computing device, such as an ECU of a concrete mixer.

In some embodiments, prior to the method being performed, a zeroing procedure is undertaken. The zero procedure comprises, for each of a plurality of drum rotation speeds: rotating the mixing drum without the concrete mix present; and determine, using the machine-learned rotation power model, a rotation power for the mixing drum at the rotation speed. A rotation profile for the empty drum is determined based on the on the respective determined rotation powers for each rotation speed. The empty drum rotation profile may be compared to a baseline rotation profile, e.g. a rotation profile of the drum when new, to assess drum build up.

At operation 4.1, motor data comprising a state a motor driving a mixing drum, gearbox data comprising a state of a gearbox arranged between the motor and the mixing drum, and load data relating to a mass of the concrete mix in the mixing drum are received from a set of sensors coupled to the concrete mixer.

In some embodiments, the motor is an electric motor. In such embodiments, the motor data may comprise: a motor speed; an electrical current and/or electrical voltage supplied to the electric motor; and a temperature of the electric motor.

Alternatively, the electric motor may be a hydraulic motor. In such embodiments, the motor data may comprise: a motor speed; an input hydraulic fluid pressure; an output hydraulic fluid pressure; and a hydraulic fluid temperature.

The gearbox data may comprise: an input torque to the gearbox; a motor or drum speed; a gearbox temperature; and a gearbox fluid level.

The load data may comprise a combined mass of the mixing drum and concrete mix. Alternatively, where a zeroing or calibration procedure is used, the load data may be the mass of the concrete mix, for example obtained by subtracting a known mass of the drum (including any concrete build-up) from the combined mass of the drum and the concrete mix.

In some embodiments, a target slump value may also be received, e.g. a user input target slump value.

At operation 4.2, an initial rotation power of the motor is determined from the motor data by a machine-learned rotation power model. The motor data is input into the model, which processes it to determine the initial rotation power.

The machine-learned rotation power model may be a neural network model, such as a fully connected neural network model. The input layer of the neural network may comprise a plurality of input neurones, one for each piece of motor data. The neural network may comprise one or more hidden layers, followed by an output layer.

The machine-learned rotation power model may have been trained using a set of training data comprising a plurality of training examples, each comprising a set of motor data and a corresponding ground-truth rotation power. The training data may have been derived from a motor test rig comprising a test motor and a plurality of sensors for determining the motor data (e.g. an ammeter, a voltmeter, a thermometer etc.) and the ground truth rotation power (e.g. a torque sensor, such as a high accuracy torque sensor).

To train the machine-learned rotation power model, training examples are input into the machine-learned rotation power model and processed according to current values of parameters (e.g. weights and biases) of the neural network to generate a candidate rotation power. The candidate rotation power is compared to the ground truth rotation power, for example using a loss/objective function (e.g. an L1 or L2 loss). The parameters of the neural network are updated based on the comparison with the aim of reducing the difference between the candidate rotation power and the ground truth rotation power. The optimisation routine may, for example, be stochastic gradient descent.

At operation 4.3, a gearbox efficiency is determined from the gearbox data using a gearbox efficiency model.

The gearbox efficiency model may be an experimentally determined control surface mapping the gearbox data to a gearbox efficiency. Alternatively, the gearbox efficiency model may be in the form of a look-up table.

In some embodiments, the gearbox efficiency model may be a machine-learned model, such as a neural network. For example, the gearbox efficiency model may be a fully connected neural network, such as the network described in relation to the machine-learned rotation power model.

At operation 4.4, an adjusted rotation power is determined by adjusting the initial rotation power of the motor of the motor based on the gearbox efficiency. The adjusted rotation power may be obtained by multiplying the initial rotation power by the gearbox efficiency.

Operations 4.1 to 4.4 may be performed for each of one or more (e.g. a plurality) of drum rotation speeds to determine a set of rotation power data. The rotation power data may comprise a plurality of drum speed-rotation power pairs.

At operation 4.5, a slump value of the concrete mix in the mixing drum is determined from the load data and the adjusted rotation power using a slump prediction model. In some embodiments, the model may use a set of rotation power data comprising a plurality of drum speed-rotation power pairs.

The slump prediction model may be an experimentally determined control surface mapping the rotation power and properties of the concrete mix (e.g. mass) to a slump value. Alternatively, the slump prediction model may be in the form of a look-up table.

In some embodiments, the slump prediction model may be a machine-learned model, such as a neural network. For example, the gearbox slump prediction may be a fully connected neural network, such as the network described in relation to the machine-learned rotation power model.

In embodiments where a zeroing procedure is used, the method may further comprise adjusting the initial rotation power of the motor based on the empty drum rotation profile prior to input into the slump prediction model.

In embodiments where a target slump is received, a hydration model may be used to determine an amount of water and/or plasticizer to add to the concrete mix to achieve the target slump. Output of this data from the hydration model may cause the concrete mixer to add the determined amount(s) to the concrete mix, for example automatically adding the amount of water to the mix from a reservoir connected to the drum.

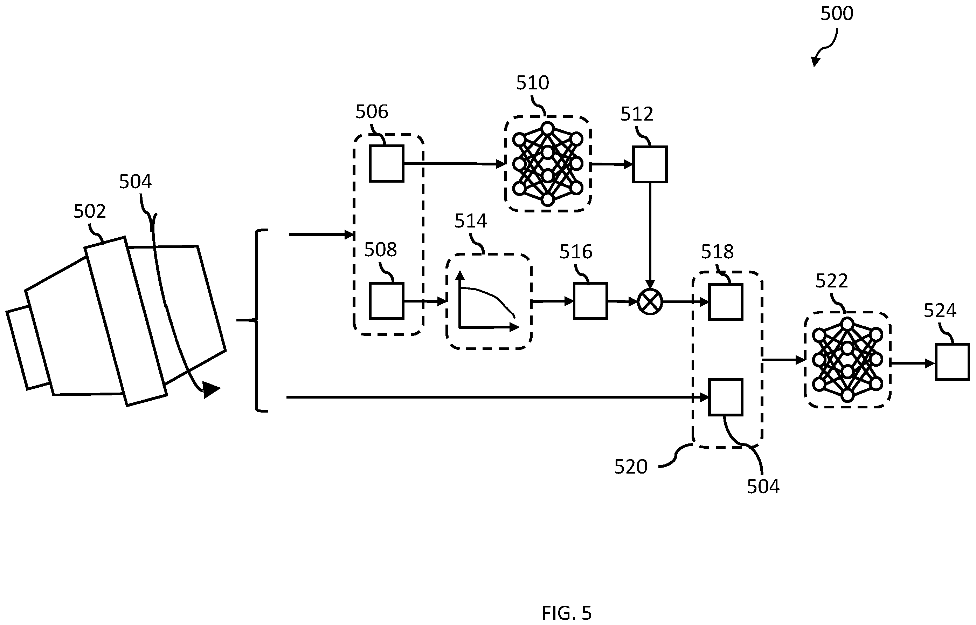

FIG. 5 shows a schematic overview of a method 500 for monitoring a drum 502 of a concrete mixer. The method may be performed by the system used to determine the slump of a concrete mix, and may reuse many of the methods described above in relation to FIG.s 1 to 4.

The drum 502 is emptied of wet concrete, then weighed to obtain an empty drum mass (i.e. the mass of the drum plus the mass of the dried concrete in the drum). Since the distribution of the concrete build-up in the drum important for energy use considerations, the mass of the concrete build-up by itself may not be enough to determine whether the drum requires maintenance.

The drum is then rotated at a plurality of drum rotation speeds 504, which may either be a sequence of steady speeds (for rotation drag calculations) or an acceleration from a low drum speed to a high drum speed (for inertia calculations), during which motor data 506 and gearbox data 508 are collected using the appropriate sensors (as described above in relation to the slump measurement method).

For each rotation speed 504, the motor data 506 is used by a machine-learned rotation power model 510 to determine an initial rotation power 512 for the motor driving the drum and the gearbox data 508 is used by a gearbox model 514 to determine a gearbox efficiency 516, as described above in relation to the slump measurement method. An updated rotation power 518 is obtained by adjusting the initial rotation power 512 based on the determined gearbox efficiency 516, again as described above in relation to the slump measurement method. The result is a set of rotation power data comprising a plurality of rotation speed - rotation power pairs 520.

A build-up model 522 uses the rotation power data 520 to determine a build-up state/build-up data 524 of the concrete mixing drum. The model 522 may, for example be a machine-learned model (i.e. an AI model), as described in more detail below. Alternatively, the model 522 may be a look-up table or control surface curve.

The build-up state/data 524 provides an indication of the amount of build-up in the drum and/r the effects of the build-up. It may, for example, comprise an estimate of the energy consumption. This may be an absolute value or an increase relative to a clean/new drum. Alternatively or additionally, the build-up state/data 524 may comprise a payload reduction when compared to a clean/new drum, i.e. an amount (e.g. mass/volume) of concrete. Alternatively or additionally, the build-up state/data 524 may comprise a rotational drag of the concrete drum or an increase in the rotational drag of the concrete drum when compared to a clean/new drum. Alternatively or additionally, the build-up state/data 524 may comprise a mass of the concrete build-up.

In some implementations, the build-up state 524 may be stored in a memory to maintain a historic record of build-up states. The stored data may be analysed periodically to determine long-term trends.

In some embodiments, a baseline profile corresponding to a clean/new drum may be generated, for example at a factory before the first use of the concrete mixer/drum. The baseline profile may be stored in a memory of a computer system associated with the concrete mixer, e.g. the ECU, and used to make comparisons to the data generated by the build-up model. When determining an initial baseline profile for a mixer/drum, the baseline profile may be compared to baseline profiles of similar mixers (e.g. mixers/drums of the same or equivalent types) to ensure the baseline performance is within tolerance. This scan effectively acts as an additional form of quality control.

The build-up model 522 may be a machine-learned model, such as a neural network. The neural network may be a fully connected neural network, a convolutional neural network or a recurrent neural network (e.g. a long short-term memory network, LSTM). The latter are useful for analysing sequences of data, such as the rotation power data.

The machine-learned build-up model 522 may be trained in a supervised manner on training data comprising rotation power data and corresponding known build-up states/data. The method described in relation to FIG. 3 may be used to train the build-up model.

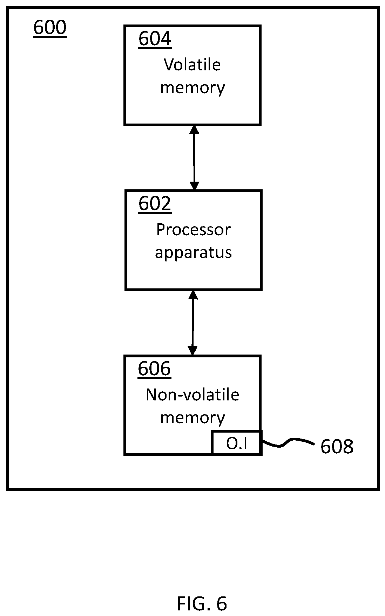

Figure 6 shows a schematic example of a system/apparatus 600 for performing any of the methods described herein. The system/apparatus shown is an example of a computing device. The system/apparatus 600 may form at least a part of a concrete mixer, e.g. part of an ECU of a concrete mixer.

The apparatus (or system) 600 comprises one or more processors 602. The one or more processors control operation of other components of the system/apparatus 600. The one or more processors 602 may, for example, comprise a general-purpose processor. The one or more processors 602 may be a single core device or a multiple core device. The one or more processors 602 may comprise a Central Processing Unit (CPU) or a graphical processing unit (GPU). Alternatively, the one or more processors 602 may comprise specialised processing hardware, for instance a RISC processor or programmable hardware with embedded firmware. Multiple processors may be included.

The system/apparatus comprises a working or volatile memory 604. The one or more processors may access the volatile memory 604 in order to process data and may control the storage of data in memory. The volatile memory 604 may comprise RAM of any type, for example, Static RAM (SRAM) or Dynamic RAM (DRAM), or it may comprise Flash memory, such as an SD-Card.

The system/apparatus comprises a non-volatile memory 606. The non-volatile memory 606 stores a set of operation instructions 508 for controlling the operation of the processors 602 in the form of computer readable instructions. The non-volatile memory 606 may be a memory of any kind such as a Read Only Memory (ROM), a Flash memory or a magnetic drive memory.

The one or more processors 602 are configured to execute operating instructions 608 to cause the system/apparatus to perform any of the methods described herein. The operating instructions 608 may comprise code (i.e. drivers) relating to the hardware components of the system/apparatus 600, as well as code relating to the basic operation of the system/apparatus 600. Generally speaking, the one or more processors 602 execute one or more instructions of the operating instructions 608, which are stored permanently or semi-permanently in the non-volatile memory 606, using the volatile memory 604 to store temporarily data generated during execution of said operating instructions 608.

Any mentioned apparatus and/or other features of particular mentioned apparatus may be provided by apparatus arranged such that they become configured to carry out the desired operations only when enabled, e.g. switched on, or the like. In such cases, they may not necessarily have the appropriate software loaded into the active memory in the non-enabled (e.g. switched off state) and only load the appropriate software in the enabled (e.g. on state). The apparatus may comprise hardware circuitry and/or firmware. The apparatus may comprise software loaded onto memory. Such software/computer programs may be recorded on the same memory/processor/functional units and/or on one or more memories/processors/ functional units.

Any mentioned apparatus/circuitry/elements/processor may have other functions in addition to the mentioned functions, and that these functions may be performed by the same apparatus/circuitry/elements/processor. One or more disclosed aspects may encompass the electronic distribution of associated computer programs and computer programs (which may be source/transport encoded) recorded on an appropriate carrier (e.g. memory, signal).

Any "computer" described herein can comprise a collection of one or more individual processors/processing elements that may or may not be located on the same circuit board, or the same region/position of a circuit board or even the same device. In some examples one or more of any mentioned processors may be distributed over a plurality of devices. The same or different processor/processing elements may perform one or more functions described herein.

The term "signalling" may refer to one or more signals transmitted as a series of transmitted and/or received electrical/optical signals. The series of signals may comprise one, two, three, four or even more individual signal components or distinct signals to make up said signalling. Some or all of these individual signals may be transmitted/received by wireless or wired communication simultaneously, in sequence, and/or such that they temporally overlap one another.

With reference to any discussion of any mentioned computer and/or processor and memory (e.g. including ROM, CD-ROM etc.), these may comprise a computer processor, Application Specific Integrated Circuit (ASIC), field-programmable gate array (FPGA), and/or other hardware components that have been programmed in such a way to carry out the inventive function.

## Claims

1. A method (200) for predicting a slump value of a concrete mix in a mixing drum of a concrete mixer, **characterised in that** the method comprising:
receiving, from one or more sensors, motor data (206) comprising a state a motor driving the mixing drum, gearbox data (212) comprising a state of a gearbox arranged between the motor and the mixing drum, and load data relating to a mass of the concrete mix in the mixing drum;
determining, by a machine-learned rotation power model (202), an initial rotation power (208) of the motor from the motor data;
determining, by a gearbox efficiency model (210), a gearbox efficiency (214) from the gearbox data;
determining an adjusted rotation power by adjusting the initial rotation power of the motor of the motor based on the gearbox efficiency; and
determining, by a slump prediction model (218), an estimated slump (204) value of the concrete mix in the mixing drum from the load data and the adjusted rotation power.

2. The method of claim 1, further comprising performing a zeroing procedure, the zeroing procedure comprising:
for each of a plurality of rotation speeds:
rotating the mixing drum without the concrete mix present; and
determining, using the machine-learned rotation power model, a rotation power for the mixing drum at the rotation speed; and
determining an empty drum rotation profile based on the respective determined rotation powers for each rotation speed.

3. The method of claim 2, further comprising adjusting the initial rotation power of the motor based on the empty drum rotation profile prior to input into the slump prediction model and/or comparing the empty drum rotation profile to a baseline rotation profile to assess drum build up.

4. The method of any preceding claim, wherein the gearbox efficiency model and/or slump prediction model comprise one or more of: a machine-learned model; a neural network; a lookup table; or a control surface.

5. The method of any preceding claim, further comprising:
receiving a target slump value for the concrete mix; and
determining, by a hydration model, an amount of water and/or plasticizer to add to the concrete mix to achieve the target slump based at least in part on the target slump value, the estimated slump value and the load data.

6. The method of claim 5, further comprising causing the concrete mixer to add the determined amount of water and/or plasticizer to the concrete mix.

7. A method (500) for monitoring concrete build up in a mixing drum (502), **characterised in that** the method comprising:
receiving, from one or more sensors, drum mass data (502) comprising a current mass of the mixing drum when empty;
for each of a plurality of drum rotation speeds (504):
receiving, from one or more sensors, motor data (506)comprising a state a motor driving the mixing drum and gearbox data (508) comprising a state of a gearbox arranged between the motor and the mixing drum;
determining, by a machine-learned rotation power model (510), an initial rotation power (512) of the motor from the motor data;
determining, by a gearbox efficiency model (514), a gearbox efficiency (516) from the gearbox data; and
determining an adjusted rotation power (518) for the drum rotation speed by adjusting the initial rotation power of the motor of the motor based on the gearbox efficiency; and
determining, using a build-up model (522), a drum build-up state (524) of the concrete mixer from the adjusted rotation powers for the plurality of drum rotation speeds.

8. The method of claim 7, wherein the drum build-up state comprises: a rotational drag of the mixing drum; a mass of concrete build-up in the mixing drum; an energy consumption increase associated with concrete build-up in the mixing drum; and/or payload reduction caused by concrete build-up in the mixing drum.

9. The method of any preceding claim, wherein the motor is an electric motor, and wherein the motor data comprises:
a motor speed;
an electrical current and/or electrical voltage supplied to the electric motor; and
a temperature of the electric motor.

10. The method of claims 1 to 8, wherein the motor is a hydraulic motor, and wherein the motor data comprises:
a motor speed;
an input fluid pressure;
an output fluid pressure; and
a fluid temperature.

11. The method of any preceding claim, wherein the gearbox data comprises:
an input torque;
a motor or drum speed;
a gearbox temperature; and
a gearbox fluid level.

12. The method of any preceding claim, wherein the machine-learned rotation power model comprises a neural network.

13. A system comprising:
a mixing drum (104) coupled to a motor (106) via a gearbox (108);
a plurality of sensors (110);
one or more processors (602); and
a memory (606),
wherein the memory stores computer readable instructions that, when executed by the one or more processors, cause the system to perform a method according to any preceding claim.

14. A concrete mixer truck (102) comprising the system of claim 13.

15. A computer program product comprising computer readable instructions that, when executed by the system of any of claims 13 or 14, cause the system to perform the method of any of claim 1-12.

## Patentansprüche

1. Verfahren (200) zum Vorhersagen eines Setzmaßwerts einer Betonmischung in einer Mischtrommel eines Betonmischers, **dadurch gekennzeichnet, dass** das Verfahren Folgendes umfasst:
Empfangen, von einem oder mehreren Sensoren, von Motordaten (206), die einen Zustand eines Motors, der die Mischtrommel antreibt, umfassen, von Getriebedaten (212), die einen Zustand eines Getriebes, das zwischen dem Motor und der Mischtrommel angeordnet ist, umfassen, und von Lastdaten, die sich auf eine Masse der Betonmischung in der Mischtrommel beziehen;
Bestimmen, durch ein maschinell erlerntes Rotationsleistungsmodell (202), einer anfänglichen Rotationsleistung (208) des Motors aus den Motordaten;
Bestimmen, durch ein Getriebewirkungsgradmodell (210), eines Getriebewirkungsgrads (214) aus den Getriebedaten;
Bestimmen einer angepassten Rotationsleistung durch Anpassen der anfänglichen Rotationsleistung des Motors des Motors basierend auf dem Getriebewirkungsgrad; und
Bestimmen, durch ein Setzmaßvorhersagemodell (218), eines geschätzten Werts für das Setzmaß (204) der Betonmischung in der Mischtrommel aus den Lastdaten und der angepassten Rotationsleistung.

2. Verfahren nach Anspruch 1, ferner umfassend Durchführen eines Nullungsvorgangs, wobei der Nullungsvorgang Folgendes umfasst:
für jede einer Vielzahl von Rotationsgeschwindigkeiten:
Rotierenlassen der Mischtrommel, ohne dass die Betonmischung vorhanden ist; und
Bestimmen, unter Verwendung des maschinell erlernten Rotationsleistungsmodells, einer Rotationsleistung für die Mischtrommel bei der Rotationsgeschwindigkeit; und
Bestimmen eines Leertrommelrotationsprofils basierend auf den jeweiligen bestimmten Rotationsleistungen für jede Rotationsgeschwindigkeit.

3. Verfahren nach Anspruch 2, ferner umfassend Anpassen der anfänglichen Rotationsleistung des Motors basierend auf dem Leertrommelrotationsprofil vor einer Eingabe in das Setzmaßvorhersagemodell und/oder Vergleichen des Leertrommelrotationsprofils mit einem Ausgangswertrotationsprofil, um eine Trommelanhaftung zu bewerten.

4. Verfahren nach einem vorhergehenden Anspruch, wobei das Getriebewirkungsgradmodell und/oder das Setzmaßvorhersagemodell eines oder mehrere von Folgendem umfassen: ein maschinell erlerntes Modell; ein neuronales Netzwerk; eine Nachschlagetabelle; oder eine Steueroberfläche.

5. Verfahren nach einem vorhergehenden Anspruch, ferner umfassend:
Empfangen eines Zielsetzmaßwerts für die Betonmischung; und
Bestimmen, durch ein Hydratationsmodell, einer Menge an Wasser und/oder Weichmacher, die der Betonmischung hinzuzufügen ist, um das Zielsetzmaß zu erreichen, basierend mindestens teilweise auf dem Zielsetzmaßwert, dem geschätzten Setzmaßwert und den Lastdaten.

6. Verfahren nach Anspruch 5, ferner umfassend Bewirken, dass der Betonmischer die bestimmte Menge an Wasser und/oder Weichmacher zu der Betonmischung hinzufügt.

7. Verfahren (500) zum Überwachen von Betonanhaftung in einer Mischtrommel (502), **dadurch gekennzeichnet, dass** das Verfahren Folgendes umfasst:
Empfangen, von einem oder mehreren Sensoren, von Trommelmassendaten (502), die eine aktuelle Masse der Mischtrommel im leeren Zustand umfassen;
für jede einer Vielzahl von Trommelrotationsgeschwindigkeiten (504):
Empfangen, von einem oder mehreren Sensoren, von Motordaten (506), die einen Zustand eines Motors, der die Mischtrommel antreibt, umfassen, und von Getriebedaten (508), die einen Zustand eines Getriebes, das zwischen dem Motor und der Mischtrommel angeordnet ist, umfassen;
Bestimmen, durch ein maschinell erlerntes Rotationsleistungsmodell (510), einer anfänglichen Rotationsleistung (512) des Motors aus den Motordaten;
Bestimmen, durch ein Getriebewirkungsgradmodell (514), eines Getriebewirkungsgrads (516) aus den Getriebedaten; und
Bestimmen einer angepassten Rotationsleistung (518) für die Trommelrotationsgeschwindigkeit durch Anpassen der anfänglichen Rotationsleistung des Motors des Motors basierend auf dem Getriebewirkungsgrad; und
Bestimmen, unter Verwendung eines Anhaftungsmodells (522), eines Trommelanhaftungszustands (524) des Betonmischers aus den angepassten Rotationsleistungen für die Vielzahl von Trommelrotationsgeschwindigkeiten.

8. Verfahren nach Anspruch 7, wobei der Trommelanhaftungszustand Folgendes umfasst: einen Rotationswiderstand der Mischtrommel; eine Masse einer Betonanhaftung in der Mischtrommel; einen Energieverbrauchsanstieg im Zusammenhang mit Betonanhaftung in der Mischtrommel; und/oder Nutzlastverringerung, die durch Betonanhaftung in der Mischtrommel verursacht ist.

9. Verfahren nach einem vorhergehenden Anspruch, wobei der Motor ein Elektromotor ist und wobei die Motordaten Folgendes umfassen:
eine Motorgeschwindigkeit;
einen elektrischen Strom und/oder eine elektrische Spannung, die dem Elektromotor zugeführt werden; und
eine Temperatur des Elektromotors.

10. Verfahren nach den Ansprüchen 1 bis 8, wobei der Motor ein Hydraulikmotor ist und wobei die Motordaten Folgendes umfassen:
eine Motorgeschwindigkeit;
einen Eingangsfluiddruck;
einen Ausgangsfluiddruck; und
eine Fluidtemperatur.

11. Verfahren nach einem vorhergehenden Anspruch, wobei die Getriebedaten Folgendes umfassen:
ein Eingangsdrehmoment;
eine Motor- oder Trommelgeschwindigkeit;
eine Getriebetemperatur; und
einen Getriebefluidstand.

12. Verfahren nach einem vorhergehenden Anspruch, wobei das maschinell erlernte Rotationsleistungsmodell ein neuronales Netzwerk umfasst.

13. System, umfassend:
eine Mischtrommel (104), die über ein Getriebe (108) an einen Motor (106) gekoppelt ist;
eine Vielzahl von Sensoren (110);
einen oder mehrere Prozessoren (602); und
einen Speicher (606),
wobei der Speicher computerlesbare Anweisungen speichert, die, wenn sie durch den einen oder die mehreren Prozessoren ausgeführt werden, das System veranlassen, ein Verfahren nach einem vorhergehenden Anspruch durchzuführen.

14. Betontransportmischer (102), umfassend das System nach Anspruch 13.

15. Computerprogrammprodukt, umfassend computerlesbare Anweisungen, die, wenn sie durch das System nach einem der Ansprüche 13 oder 14 ausgeführt werden, das System veranlassen, das Verfahren nach einem der Ansprüche 1-12 durchzuführen.

## Revendications

1. Procédé (200) de prédiction d'une valeur d'affaissement d'un mélange de béton dans un tambour de mélange d'un mélangeur de béton, **caractérisé en ce que** le procédé comprend :
la réception, en provenance d'un ou plusieurs capteurs, de données de moteur (206) comprenant un état d'un moteur entraînant le tambour de mélange, de données de boîte de vitesses (212) comprenant un état d'une boîte de vitesses disposée entre le moteur et le tambour de mélange, et de données de charge relatives à une masse du mélange de béton dans le tambour de mélange ;
la détermination, par un modèle de puissance de rotation par apprentissage automatique (202), d'une puissance de rotation initiale (208) du moteur à partir des données de moteur ;
la détermination, par un modèle d'efficacité de boîte de vitesses (210), d'une efficacité de boîte de vitesses (214) à partir des données de boîte de vitesses ;
la détermination d'une puissance de rotation ajustée en ajustant la puissance de rotation initiale du moteur du moteur sur la base de l'efficacité de boîte de vitesses ; et
la détermination, par un modèle de prédiction d'affaissement (218), d'une valeur d'affaissement estimée (204) du mélange de béton dans le tambour de mélange à partir des données de charge et de la puissance de rotation ajustée.

2. Procédé selon la revendication 1, comprenant en outre la réalisation d'une procédure de remise à zéro, la procédure de remise à zéro comprenant :
pour chacune d'une pluralité de vitesses de rotation :
la rotation du tambour de mélange sans présence du mélange de béton ; et
la détermination, à l'aide du modèle de puissance de rotation par apprentissage automatique, d'une puissance de rotation pour le tambour de mélange à la vitesse de rotation ; et
la détermination d'un profil de rotation de tambour vide sur la base des puissances de rotation déterminées respectives pour chaque vitesse de rotation.

3. Procédé selon la revendication 2, comprenant en outre l'ajustement de la puissance de rotation initiale du moteur sur la base du profil de rotation de tambour vide avant l'entrée dans le modèle de prédiction d'affaissement et/ou la comparaison du profil de rotation de tambour vide à un profil de rotation de référence pour évaluer l'accumulation de tambour.

4. Procédé selon l'une quelconque des revendications précédentes, dans lequel le modèle d'efficacité de boîte de vitesses et/ou le modèle de prédiction d'affaissement comprennent un ou plusieurs éléments parmi : un modèle d'apprentissage automatique ; un réseau neuronal ; une table de consultation ; ou une surface de contrôle.

5. Procédé selon l'une quelconque des revendications précédentes, comprenant en outre :
la réception d'une valeur d'affaissement cible pour le mélange de béton ; et
la détermination, par un modèle d'hydratation, d'une quantité d'eau et/ou de plastifiant à ajouter au mélange de béton pour atteindre l'affaissement cible sur la base au moins en partie de la valeur d'affaissement cible, de la valeur d'affaissement estimée et des données de charge.

6. Procédé selon la revendication 5, comprenant en outre le fait d'amener le mélangeur de béton à ajouter la quantité déterminée d'eau et/ou de plastifiant au mélange de béton.

7. Procédé (500) de surveillance de l'accumulation de béton dans un tambour de mélange (502), **caractérisé en ce que** le procédé comprend :
la réception, en provenance d'un ou de plusieurs capteurs, de données de masse de tambour (502) comprenant une masse actuelle du tambour de mélange lorsqu'il est vide ;
pour chacune d'une pluralité de vitesses de rotation du tambour (504) :
la réception, en provenance d'un ou plusieurs capteurs, de données de moteur (506) comprenant un état d'un moteur entraînant le tambour de mélange et de données de boîte de vitesses (508) comprenant un état d'une boîte de vitesses disposée entre le moteur et le tambour de mélange ;
la détermination, par un modèle de puissance de rotation par apprentissage automatique (510), d'une puissance de rotation initiale (512) du moteur à partir des données du moteur ;
la détermination, par un modèle d'efficacité de boîte de vitesses (514), d'une efficacité de boîte de vitesses (516) à partir des données de boîte de vitesses ; et
la détermination d'une puissance de rotation ajustée (518) pour la vitesse de rotation du tambour en ajustant la puissance de rotation initiale du moteur du moteur sur la base de l'efficacité de boîte de vitesses ; et
la détermination, à l'aide d'un modèle d'accumulation (522), d'un état d'accumulation de tambour (524) du mélangeur de béton à partir des puissances de rotation ajustées pour la pluralité de vitesses de rotation de tambour.

8. Procédé selon la revendication 7, dans lequel l'état d'accumulation du tambour comprend :
une traînée de rotation du tambour de mélange ; une masse d'accumulation de béton dans le tambour de mélange ; une augmentation de la consommation d'énergie associée à l'accumulation de béton dans le tambour de mélange ; et/ou une réduction de la charge utile causée par l'accumulation de béton dans le tambour de mélange.

9. Procédé selon l'une quelconque des revendications précédentes, dans lequel le moteur est un moteur électrique, et dans lequel les données de moteur comprennent :
une vitesse du moteur ;
un courant électrique et/ou une tension électrique fournis au moteur électrique ; et
une température du moteur électrique.

10. Procédé selon les revendications 1 à 8, dans lequel le moteur est un moteur hydraulique, et dans lequel les données de moteur comprennent :
une vitesse de moteur ;
une pression de fluide d'entrée ;
une pression de fluide de sortie ; et
une température de fluide.

11. Procédé selon l'une quelconque des revendications précédentes, dans lequel les données de boîte de vitesses comprennent :
un couple d'entrée ;
une vitesse du moteur ou du tambour ;
une température de boîte de vitesses ; et
un niveau de fluide de boîte de vitesses.

12. Procédé selon l'une quelconque des revendications précédentes, dans lequel le modèle de puissance de rotation par apprentissage automatique comprend un réseau neuronal.

13. Système comprenant :
un tambour mélangeur (104) couplé à un moteur (106) par l'intermédiaire d'une boîte de vitesses (108) ;
une pluralité de capteurs (110) ;
un ou plusieurs processeurs (602) ; et
une mémoire (606) ;
dans lequel la mémoire stocke des instructions lisibles par ordinateur qui, lorsqu'elles sont exécutées par l'un ou plusieurs processeurs, amènent le système à réaliser un procédé selon une quelconque revendication précédente.

14. Camion mélangeur de béton (102) comprenant le système selon la revendication 13.

15. Produit-programme informatique comprenant des instructions lisibles par ordinateur qui, lorsqu'elles sont exécutées par le système selon l'une quelconque des revendications 13 ou 14, amènent le système à réaliser le procédé selon l'une quelconque des revendications 1 à 12.
